# EUROPEAN PATENT SPECIFICATION

(11) **EP 4 688 101 B1**
(45) Date of publication and mention of the grant of the patent: **02.12.2026**
(21) Application number: 24712031.4
(22) Date of filing: 14.03.2024
(51) Int. Cl.: A61M 39/22, A61M 1/14, A61M 1/36, A61M 39/00

(54) **VALVE WITH SAMPLING AND PURGE PORTS**
VENTIL MIT PROBENAHME- UND SPÜLÖFFNUNGEN
VALVE AVEC ORIFICES D'ÉCHANTILLONNAGE ET DE PURGE

(30) Priority: 24.03.2023 US 202363491980 P
(43) Date of publication of application: 11.02.2026
(73) Proprietor: MAQUET Cardiopulmonary GmbH, 76437 Rastatt (DE)
(72) Inventor: KORELL, Andreas, 72336 Balingen (DE)
(74) Representative: Zacco GmbH
(86) International application number: PCT/EP2024/056817
(87) International publication number: WO 2024/200026

(56) References cited:
- EP-A1- 0 331 526
- JP-A- H10 314 298
- US-A- 4 210 178
- US-A- 4 414 999
- US-A1- 2002 000 253
- US-A1- 2014 228 815

## Description

### BACKGROUND OF THE DISCLOSURE

### Field of the Disclosure

The present disclosure relates to valves for controlling fluid flow, and, more particularly to valves with sampling and/or purge ports.

### Description of Related Art

Extracorporeal blood circulation is a critical component of modern medical care. Various types of extracorporeal blood circulation systems, such as extracorporeal membrane oxygenation (ECMO) machines, heart-lung machines, cardiopulmonary bypass machines, pump-assisted lung protection machines, and dialysis machines, may be used to perform cardiac and/or respiratory support to an individual whose own heart and/or lungs are/is unable to sustain life. During installation of such systems, connection of such systems to a patient, and throughout the course of operation, access to the fluid circuits of the systems may be required or desirable to perform various functions. For example, fluid circuit may need to be purged of air to prevent air injection into the patient. In some instances, it may be necessary or desirable to draw blood from the fluid circuit for analysis. These and other functions may be performed via valves that can be opened and closed to provide selective access to the fluid circuits.

US 4 414 999 A discloses a flow regulating assembly and components are provided for a catheter system. The assembly provides a regulated flow of medical fluid with an intermittent operator-controlled large-volume flushing flow. The assembly includes a flow-occluding piston reciprocal within a housing to move from the regulated flow position to a position completely out of the path of fluid flow from the inlet to the outlet. Actuation of the piston is from the exterior of the housing, and adjustment of the regulated flow volume is also effected from the housing exterior. The assembly components may be sterilized and fitted together in a clean room without welding or gluing being necessary.

JP H10 314298 A discloses a branch pipe for an external circulating circuit, wherein when blood is newly passed through a sub-passage, an opening part is released by pulling up an opening/closing member from a closing position to a retreat position, and blood in a main passage is introduced into the sub-passage. To stop blood from the main passage from entering the sub-passage, the opening/closing member is returned to the closing position from the retreat position. In the branch pipe for an external circulating circuit, when there is no need to branch off the blood, the opening/closing member is inserted/arranged in an opening/closing passage.

EP 0 331 526 A1 discloses a resilient valve core, biased against its seat in a molded body, controls large flow volumes for flushing. A small resilient tube embedded in the core bypasses the valve to provide smaller flow volumes for IV-fluid drip. For flushing, the core is deformed, rather than bodily moved, relative to the body. A fluid-flow channel is recessed along one side of the valve cavity, and the valve seat forms a short barrier across this channel. The core is a T-shaped unitary member; the center of the "upper" surface of the T crossbar is biased against the seat, and the extrema of the crossbar and hermetically sealed against the valve body, while the stem of the T extends outward from the body. A user squeezes a cowling mounted outside the body to start and control flushing. The cowling itself deforms to pull the stem of the T outward, deforming the T and separating the middle of its crossbar portion from the valve seat. A user can elect to grasp and pull the stem directly. Parts of the upper side of the extrema of the T crossbar are relieved, forming a smooth fluid-flow transition with the inlet and outlet. These paths, the relieved parts of the crossbar, and the cavity interior are all smooth, gently tapering and well fitted, forming nearly an in-line flush path to minimize gas-bubble trapping.

US 4 210 178 A discloses a flushing device for placement in a slow infusion line of liquid to a human or animal body through a catheter, cannula or similar item that provides a flushing or cleaning by-pass route merely by, and only during, finger pressure applied to the flushing actuator.

US 2002/000253 A1 discloses a valve apparatus that includes a housing having proximal and distal ends and defining a passageway therethrough. An access port of the housing defines a bore configured for selective communication with the passageway. The valve apparatus includes a hollow insert which is disposed in the distal end of the passageway and includes a portion that is disposed beneath the bore defined by the access port. The housing additionally includes retention tabs on the distal end that engage corresponding openings in a fitting that is attached to the housing and includes a nipple slidingly received within the insert. A seat on the nipple contacts a flange of the insert to form a substantially leak proof connection. A substantially identical fitting engages corresponding retention tabs on the access port and is configured to slidingly receive a plunger, disposed in the bore, within a passage defined by the nipple. A sealing ring disposed in the bore around the plunger is compressed by the nipple and acts to prevent leakage past the plunger. The plunger is biased upwards out of the passageway, defined by the housing, by the resilience of the insert, so that fluid can readily flow between the proximal and distal ends of the passageway. Attachment of a medical device to the fitting on the access port pushes the plunger downward so that a flushing passage defined by the plunger establishes fluid communication between the medical device and the proximal end of the passageway, thereby permitting introduction of material from the medical device into the proximal end of the passageway, and to another medical device in fluid communication therewith. Upon detachment of the medical device, the resilience of the insert moves the plunger upward in the bore so that fluid communication between the flushing passage and the proximal end of the passageway is disestablished, and fluid communication between the proximal and distal ends of the passageway is reestablished.

US 2014/228815 A1 discloses a connector system for use in connecting conduits together, such as those used with an extracorporeal blood circulation system. The system has a male and female connector and a seal. The seal is operatively associated with the male and female connectors and forms a fluid tight connection between the male and female connectors. An inner surface of the seal and the inner surfaces of the first and second coupling ends form a continuous and smooth transitional surface.

While conventional valves may be able to provide the necessary access to fluid circuits, they also have inherent disadvantages that may pose risks to patients. In particular, the structure of conventional valves includes obstruction which blood impacts when flowing through the valve. Conventional valves have increased hemolysis through edges/corners resulting from an integration of a port in the blood path. Conventional valves also have increased risk of clotting of blood resulting from "dead zones" in the blood path in which the blood is stagnant in the "normal" situation when the valve is closed. Convenitonal purge ports also include purge ports that are press fit into the housing. Thus, sterilization gases cannot reach parts of the stop cock or purge port, which are later, in the application, exposed to blood. Such impacts can rupture red blood cells, leading to conditions such as hemolysis. Further, obstructions in conventional valves may cause unwanted coagulation of blood in the valve.

### SUMMARY OF THE DISCLOSURE

In view of the foregoing, there exists a need for valves for use in extracorporeal circulation systems which allow for sampling, purging, and like functions, while eliminating negative effects on the blood. Accordingly, embodiments of the present disclosure are directed a valve for use in an extracorporeal blood circulation system. The valve includes a valve port defining a valve lumen, an access port extending at an angle from the valve lumen and defining an access lumen in fluid communication with the valve lumen, and a valve element disposed in the valve port. The valve element is movable between a closed position in which the valve element engages a sidewall of the valve lumen to prevent fluid flow through the access lumen, and an open position in which the valve element is retracted relative to the closed position to allow fluid to flow through the valve lumen and into the access lumen.

In some embodiments, the valve port has a tapered sidewall narrowing in a direction toward the main lumen, and the valve element includes a tapered pin configured to seal against the tapered sidewall in the closed position.

In some embodiments, the tapered sidewall has a taper angle in a range of 3° to 15°.

In some embodiments, the tapered sidewall acts as a stop preventing the tapered pin from protruding into the main lumen.

In some embodiments, in the closed position of the valve element, a distal end of the valve element is substantially flush with an inner sidewall of a main fluid path component to which the valve may be fluidly connected.

In some embodiments, the valve element includes a knob for adjusting the valve element between the closed position and the open position.

In some embodiments, the valve port included a threaded section, and a proximal end of the valve element is threaded to engage the threaded section of the valve port.

In some embodiments, the threaded section includes a threaded insert connected to the valve port.

In some embodiments, the valve port extends at angle of approximately 90° from a main fluid path component to which the valve may be fluidly connected.

In some embodiments, the access port extends at angle from the valve port in a range of approximately 30° to approximately 90°.

In some embodiments, the access port includes a luer fitting.

In some embodiments, the valve further includes a seal disposed in the valve port for preventing fluid flow between the valve element and a sidewall of the valve port.

In some embodiments, the extracorporeal blood circulation system is selected from a group consisting of an ECMO machine, a heart-lung machine, a cardiopulmonary bypass machine, a pump-assisted lung protection machine, and a dialysis machine.

Other embodiments of the present disclosure are directed to an extracorporeal blood circulation system, including a fluid path component and a valve. The valve includes a main fluid path component defining a longitudinal axis and a main lumen extending parallel to the longitudinal axis, a valve port extending at an angle from the main lumen, the valve port defining a valve lumen in fluid communication with the main lumen, an access port extending at an angle from the valve lumen and defining an access lumen in fluid communication with the valve lumen, and a valve element disposed in the valve port. The valve element is movable between a closed position in which the valve element engages a sidewall of the valve lumen to prevent fluid flow from the main lumen to the access lumen, and an open position in which the valve element is retracted relative to the closed position to allow fluid to flow from the main lumen, through the valve lumen, and into the access lumen. The main fluid path component is disposed internally in a fluid path of the fluid path component.

In some embodiments, the fluid path component includes at least one of an oxygenator and a heat exchanger.

In some embodiments, the valve port has a tapered sidewall narrowing in a direction toward the main lumen, and the valve element includes a tapered pin configured to seal against the tapered sidewall in the closed position.

In some embodiments, wherein the tapered sidewall has a taper angle in a range of 3° to 15°.

In some embodiments, the tapered sidewall acts as a stop preventing the tapered pin from protruding into the main lumen.

In some embodiments, in the closed position of the valve element, a distal end of the valve element is substantially flush with an inner sidewall of the main fluid path component.

In some embodiments, the valve element includes a knob for adjusting the valve element between the closed position and the open position.

In some embodiments, the valve port includes a threaded section, and a proximal end of the valve element is threaded to engage the threaded section of the valve port.

In some embodiments, the threaded section includes a threaded insert connected to the valve port.

In some embodiments, the valve port extends at angle of approximately 90° from the main fluid path component.

In some embodiments, the access port extends at angle from the valve port in a range of approximately 30° to approximately 90°.

In some embodiments, the access port includes a luer fitting.

In some embodiments, the valve further includes a seal disposed in the valve port for preventing fluid flow between the valve element and a sidewall of the valve port.

In some embodiments, the extracorporeal blood circulation system is selected from a group consisting of an ECMO machine, a heart-lung machine, a cardiopulmonary bypass machine, a pump-assisted lung protection machine, and a dialysis machine.

In another embodiment, a valve for use in an extracorporeal blood circulation system, may include a valve port defining a valve lumen in fluid communication with the main lumen, a valve element disposed in the valve port, wherein the valve element defines a fluid path channel and is configured to be movable between: a closed position in which the valve element engages a sidewall of the valve lumen to prevent fluid flow through the access lumen; and an open position in which the valve element is retracted relative to the closed position to allow fluid to flow through the valve lumen and into the access lumen; and an access port formed on a proximal end of the valve element and defining an access lumen in fluid communication with the valve lumen.

In another embodiment, the access port comprises a luer lock.

In another embodiment, the fluid path channel extends from a distal end of the valve element to a proximal end of the valve element.

In another embodiment, the valve port has a tapered sidewall narrowing in a direction toward the main lumen; and wherein the valve element comprises a tapered pin configured to seal against the tapered sidewall in the closed position.

In another embodiment, the tapered sidewall has a taper angle in a range of 3° to 15°.

In another embodiment, the tapered sidewall acts as a stop preventing the tapered pin from protruding into a main path fluid component to which the valve may be fluidly connected.

In another embodiment, in the closed position of the valve element, a distal end of the valve element is substantially flush with an inner sidewall of a main fluid path component to which the valve may be fluidly connected.

In another embodiment, a seal is disposed in the valve port for preventing fluid flow between the valve element and a sidewall of the valve port.

In another embodiment, the extracorporeal blood circulation system is selected from a group consisting of an ECMO machine, a heart-lung machine, a cardiopulmonary bypass machine, a pump-assisted lung protection machine, and a dialysis machine.

Further details and advantages of the various non-limiting examples described in detail herein will become clear upon reviewing the following detailed description of the various non-limiting examples in conjunction with the accompanying drawing figures.

### BRIEF DESCRIPTION OF THE DRAWINGS

FIG. 1 is a perspective view of a valve in accordance with an embodiment of the present disclosure;
FIG. 2 is a cross-sectional view of the valve of FIG. 1, taken along line A-A, in an open position;
FIG. 3 is a cross-sectional view of the valve of FIG. 1, taken along line A-A, in a closed position;
FIG. 4 is a cross-sectional view of the valve of FIG. 1, taken along line A-A, in the open position;
FIG. 5 is a perspective view of an oxygenator assembly in accordance with an embodiment of the present disclosure.
FIG. 6 is a perspective view of a valve in accordance with an embodiment of the present disclosure with the valve in a closed position.
FIG. 7 is a perspective view of the valve of FIG. 6 in an open position.
FIG. 8 is a cross-sectional view of the valve of FIG. 6 in the closed position.
FIG. 9 is a cross-sectional view of the valve of FIG. 6 in the open position.

Referring to the drawings in which like reference characters refer to like parts throughout the several views thereof, the present disclosure is generally directed to an extracorporeal circulation system and a fluid heating pump assembly for use in such a system.

### DETAILED DESCRIPTION

For purposes of the description hereinafter, the terms "upper", "lower", "right", "left", "vertical", "horizontal", "top", "bottom", "lateral", "longitudinal", and derivatives thereof shall relate to the disclosure as it is oriented in the drawing figures. Spatial or directional terms, such as "left", "right", "inner", "outer", "above", "below", and the like, are not to be considered as limiting as the disclosed embodiments can assume various alternative orientations.

As used herein, the singular form of "a", "an", and "the" include plural referents unless the context clearly dictates otherwise.

All numbers used in the specification and claims are to be understood as being modified in all instances by the term "about". The terms "approximately", "about", and "substantially" mean a range of plus or minus ten percent of the stated value.

As used herein, the term "at least one of" is synonymous with "one or more of". For example, the phrase "at least one of A, B, and C" means any one of A, B, and C, or any combination of any two or more of A, B, and C. For example, "at least one of A, B, and C" includes one or more of A alone; or one or more of B alone; or one or more of C alone; or one or more of A and one or more of B; or one or more of A and one or more of C; or one or more of B and one or more of C; or one or more of all of A, B, and C. Similarly, as used herein, the term "at least two of" is synonymous with "two or more of". For example, the phrase "at least two of D, E, and F" means any combination of any two or more of D, E, and F. For example, "at least two of D, E, and F" includes one or more of D and one or more of E; or one or more of D and one or more of F; or one or more of E and one or more of F; or one or more of all of D, E, and F.

It is also to be understood that the specific devices and processes illustrated in the attached drawings, and described in the following specification, are simply examples of the disclosure. Hence, specific dimensions and other physical characteristics related to the examples disclosed herein are not to be considered as limiting.

The terms "first", "second", and the like are not intended to refer to any particular order or chronology, but refer to different conditions, properties, or elements.

The term "at least" is synonymous with "greater than or equal to". The term "not greater than" is synonymous with "less than or equal to".

It is to be understood that the disclosure may assume alternative variations and step sequences, except where expressly specified to the contrary. It is also to be understood that the specific devices and processes illustrated in the attached drawings, and described in the following specification, are simply exemplary aspects of the disclosure. Hence, specific dimensions and other physical characteristics related to the examples disclosed herein are not to be considered as limiting.

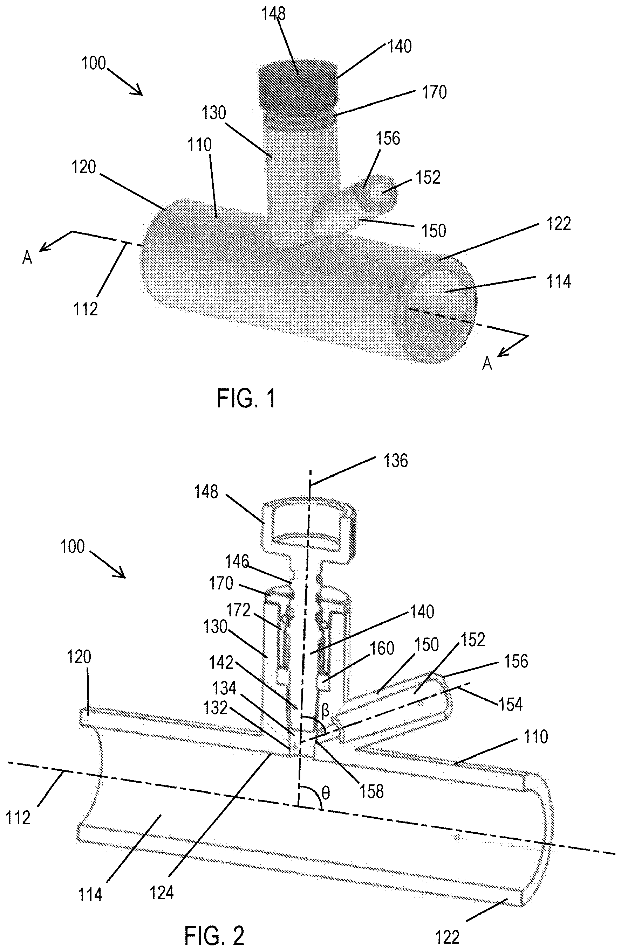

Referring now to FIGS. 1-4, the present disclosure is directed to a valve 100 for controlling flow, and particularly for allowing sampling and/or purging of fluid. The valve 100 may be used in any fluid flow system, but may be particularly suited for use in an extracorporeal blood circulation system such as an ECMO machine, a heart-lung machine, a cardiopulmonary bypass machine, a pump-assisted lung protection machine, and a dialysis machine. The valve 100 includes a main fluid path component 110 through which fluid flows within the valve 100. The main fluid path component 110 defines a longitudinal axis 112 extending from a first end 120 of the fluid path component 110 to a second end 122 of the fluid path component 110. A main lumen 114 defined by an inner sidewall 124 of the main fluid path component 110 extends parallel to the longitudinal axis 112. In use, fluid may flow through the main lumen 114 in the general direction of the longitudinal axis 112 between from the first end 120 to the second end 122, and vice versa. In some embodiments, flow through the main fluid path component 110 is bidirectional meaning that the first end 120 may serve as an inlet and/or an outlet, and, likewise, the second end 122 may serve as an inlet and/or an outlet. Thus, the valve 100 may be installed in a fluid flow system in either orientation with respect to the flow direction of fluid. Each of the first end 120 and the second end 122 of the main fluid path component 110 may include a connector, such as a luer lock, bayonet connector, slip fitting, or the like for suitable for connection to adjacent components of the system.

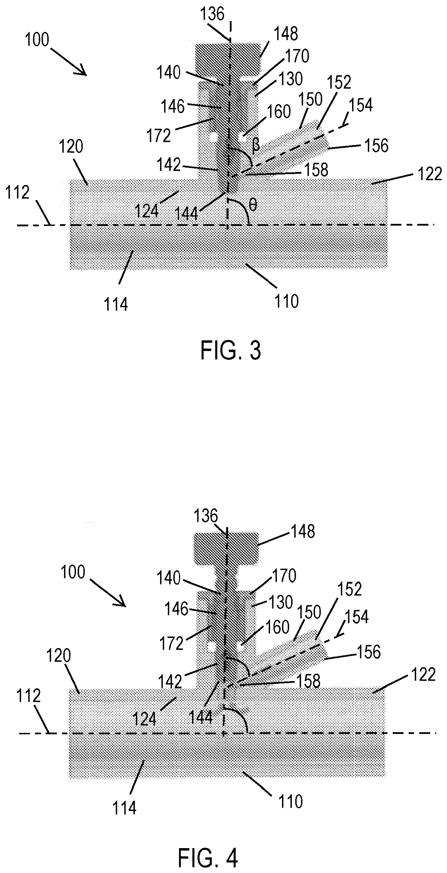

The valve 100 further includes a valve port 130 extending from the main fluid path component 110. The valve port 130 has an inner sidewall 132 defining a valve lumen 134 extending parallel to a valve axis 136. The valve lumen 134, in an open position of the valve 100, is in fluid communication with the main lumen 114 of the main fluid path component 110. An angle θ between the longitudinal axis 112 and the valve axis 136 may be approximately 90°. It is also contemplated that the valve axis 136 may be at a different angle other than 90°. A valve element 140 is disposed in the valve lumen 134 and is configured to translate along the valve axis 136. The valve element 140 may be in the form of a pin having a tapered distal end 142. The inner sidewall 132 of the valve port 130 may be tapered such that the valve lumen 134 is frusto-conical in shape. The taper of the inner sidewall 132 may be complementary to the shape of the tapered distal end 142 of the valve element 140, so that engagement between the inner sidewall 132 and the distal end 142 forms a substantially fluid-tight seal when the valve element 140 is in the closed position shown in FIG. 3.

The valve 100 further includes an access port 150 extending from the fluid path componentthe valve port 130. As will be described herein, the access port 150 may be used to take or add fluid samples from the main lumen 114 and/or to purge the main lumen 114. The access port 150 defines an access lumen 152 extending along an access axis 154. The access lumen 152, in an open position of the valve 100, is in fluid communication with the valve lumen 134. The access axis 154 may extend at an angle β from the valve axis 136, wherein the angle β in a range of approximately 20° to 70°, in some embodiments about 45°. In another example, the angle β is in the range of approximately 30° to 90°. The access port 150 may include a fitting 156, such as a luer fitting, for connection to another component such as a syringe.

The valve element 140 is movable between two states, namely a closed position illustrated in FIG. 3 and an open position illustrated in FIGS. 2 and 4. As previously described, in the closed position, the valve element 140 forms a substantially fluid-tight seal with the inner sidewall 132 of the valve port 130. In particular, the tapered distal end 142 of the valve element 140 engages the complementary taper of the inner sidewall 132 to create a substantially fluid-tight interface. The taper angles of the valve element 140 and the inner sidewall 132 may be, in some embodiments, in a range of approximately 1° to approximately 15°. In another example, the taper angle of the valve element 140 and the inner sidewall 132 may be 3° to 15°. In the closed position, the valve element 140 extends over an opening 158 between the valve lumen 134 and the access lumen 152, thereby preventing fluid from flowing from the main lumen 114 into the valve lumen 134 and ultimately to the access lumen 152. Also in the closed position, an end face 144 of the valve element 140 is substantially flush with the sidewall 124 of the main fluid path component 110 to provide a smooth transition across which fluid can flow through the main lumen 114. The taper of the inner sidewall 132 may act as a stop or limit that prevents the valve member 140 from protruding into the main lumen 114 significantly beyond the sidewall 124 of the main fluid path component 110. The flush, smooth transition between the sidewall 124 of the main fluid path component 110 and the end face 144 is free of protruding edges that could cause unwanted coagulation of the fluid (e.g. blood) flowing in the main lumen 114 and/or impacts that could potentially lead to conditions such as hemolysis.

To utilize the valve 100 for sampling or purging, the valve element 140 is moved from the closed position illustrated in FIG. 3 to the open position illustrated in FIGS. 2 and 4. In the open position, the end face 144 of the valve element 140 is retracted away from the sidewall 124 of the main lumen 114 to allow fluid flow into the valve lumen 134. The end face 144 is spaced apart from the sidewall 124 such that fluid can flow from the main lumen 114 into valve lumen 134, through the opening 158, and ultimately to the access lumen 152. With the valve element 140 retracted in this position, the fluid-tight engagement between the tapered distal end 142 and the inner sidewall 132 is no longer present, so fluid could potentially flow between the inner sidewall 132 and the valve element 140. To prevent this, one or more seals 160 (e.g. and O-rings) are disposed around the valve element 140.

Referring again to FIGS. 1-4, the valve element 140 may be threaded into the valve port 130 such the rotating the valve element 140 moves the valve element 140 along (i.e. parallel to) the valve axis 134. In particular, rotating the valve element 140 in one direction moves the valve element 140 from the open position *(see* FIGS. 2 and 4) to the closed position *(see* FIG. 3), while rotating the valve element 140 in an opposite direction moves the valve element 140 from the closed position to the open position. The valve element 140 may include a knob 148 to facilitate manually rotating the valve element 140 by a clinician.

The valve element 140 may include an external threaded portion 146 located proximally of the distal end 142. The external threaded portion 146 engages a complementary internal threaded portion 172 of the valve port 130. In particular, the internal threaded portion 172 may be provided on a threaded insert 170 disposed in the valve port 130. The threaded insert 170 may be connected to the valve port 130 via a press fit, snap fit, adhesive, weld, or the like. In addition to allowing rotation of the valve element 140, the threaded insert 170 may also retain the seal 160 in position in the valve port 130. It is also contemplated that the valve element 140 could be moved by a spring of a magnetic, electric, or pneumatic actuator.

To take a sample of the fluid within the main lumen 114, the clinician connects a syringe (or other collection device) to the fitting 156 of the outlet port 150, moves the valve element 140 to the open position *(see* FIGS. 2 and 4) so that fluid in the main lumen 114 can flow into the access lumen 152, as described above. The clinician then draws fluid into the collection device connected to the access port 150, and returns the valve element 140 to the closed position *(see* FIG. 3).

To purge the valve 100 and connected fluid components, the clinician connects a syringe (or other collection device) to the fitting 156 of the outlet port 150, moves the valve element 140 to the open position (see FIGS. 2 and 4) so that air in the main lumen 114 can flow into the access lumen 152. The clinician then draws air into the collection device connected to the access port 150, and returns the valve element 140 to the closed position (see FIG. 3) once all air has been removed.

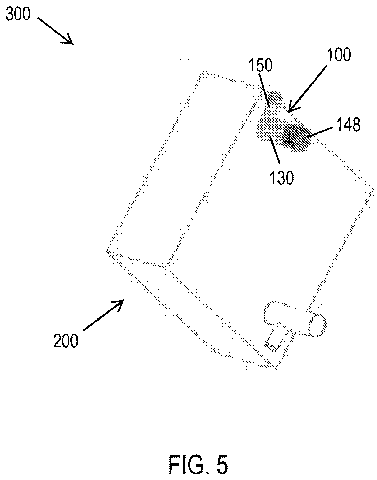

Referring now to FIG. 5, the valve 100 of the present disclosure may be attached to or integrated with another fluid path component 200 (such as an oxygenator, heat exchanger, or the like) of an extracorporeal circulation system 300. In the embodiment shown, the main fluid path component 110 is disposed internally in a fluid path of the component 200, with the access port 150 and at least the knob 148 of the valve element 140 projecting from the component 200 to allow the clinical to actuate the knob 148 and take a fluid sample 200 from the component and/or purge the component 200 via the access port 150.

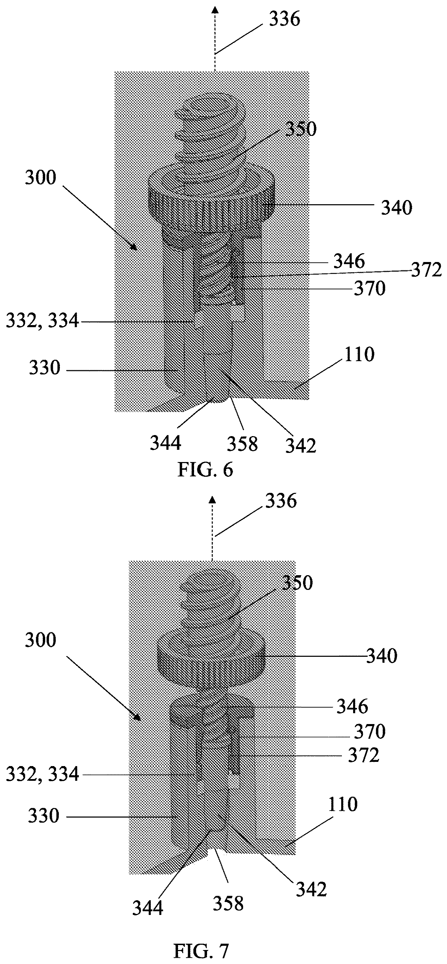

With reference to FIGS. 6-9 , in another non-limiting embodiment or aspect of the present disclosure, a valve 300 is shown and described. The valve 300 may be fluidly connected to a main fluid path component 110 similar to the valve 100 described above. The valve 300 includes a valve port 330 extending from the main fluid path component 110. The valve port 330 has an inner sidewall 332 defining a valve lumen 334 extending parallel to a valve axis 336. The valve lumen 334, in an open position of the valve 300, is in fluid communication with the main lumen of the main fluid path component 110. A valve element 340 is disposed in the valve lumen 334 and is configured to translate along the valve axis 336. The valve element 340 may be in the form of a pin having a tapered distal end 342. The inner sidewall 332 of the valve port 330 may be tapered such that the valve lumen 334 is frusto-conical in shape. The taper of the inner sidewall 332 may be complementary to the shape of the tapered distal end 342 of the valve element 340, so that engagement between the inner sidewall 332 and the distal end 342 forms a substantially fluid-tight seal when the valve element 340 is in the closed position shown in FIG. 6.

The valve 300 may include an access port 350 that is formed integral with the valve element 340. The access port 350 may be provided on a proximal end of the valve element 340. In one example, the access port 350 is a male portion of a luer lock.

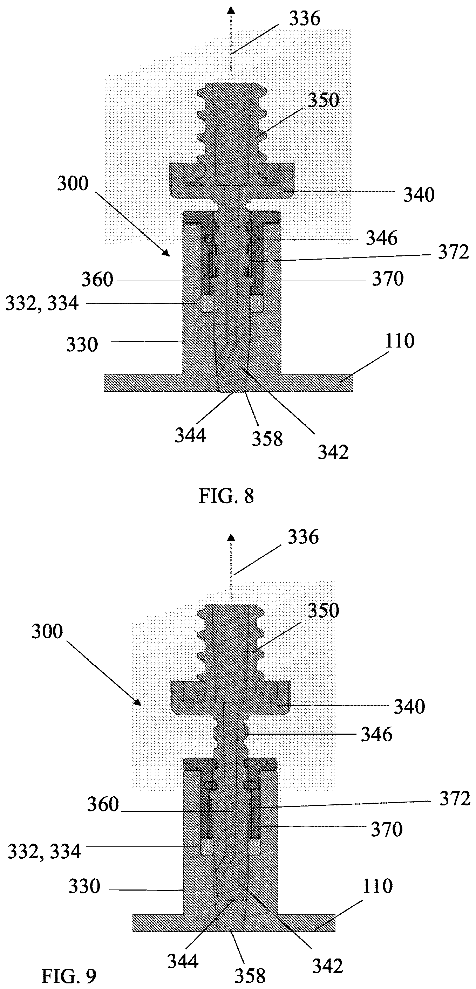

The valve element 340 is movable between two states, namely a closed position illustrated in FIGS. 6 and 8 and an open position illustrated in FIGS. 7 and 9. As previously described, in the closed position, the valve element 340 forms a substantially fluid-tight seal with the inner sidewall 332 of the valve port 330. In particular, the tapered distal end 342 of the valve element 340 engages the complementary taper of the inner sidewall 332 to create a substantially fluid-tight interface. The taper angles of the valve element 340 and the inner sidewall 332 may be, in some embodiments, in a range of approximately 1° to approximately 15°. In another example, the tager angle of the valve element 340 and the inner sidewall 332 may be 3° to 15°. In the closed position, the valve element 340 extends over an opening 358 between the valve lumen 334 and the main fluid path component 110, thereby preventing fluid from flowing from the main fluid path component 110 into the valve lumen 334 and ultimately to the access port 350. Also in the closed position, an end face 344 of the valve element 340 is substantially flush with the sidewall 124 of the main fluid path component 110 to provide a smooth transition across which fluid can flow through the main fluid path component 110. The taper of the inner sidewall 332 may act as a stop or limit that prevents the valve member 340 from protruding into the main fluid path component 110 significantly beyond the sidewall 124 of the main fluid path component 110. The flush, smooth transition between the sidewall 124 of the main fluid path component 110 and the end face 344 is free of protruding edges that could cause unwanted coagulation of the fluid (e.g. blood) flowing in the main fliud path component 110 and/or impacts that could potentially lead to conditions such as hemolysis.

**In** one non-limiting embodiment or aspect of the present disclosure, the valve element 340 may define a fluid path channel 360 that extends from an opening in the distal end 342 of the valve element 340 to an opposing opening in the proximal end of the valve element 340. When the valve element 340 is in the open position, a fluid path is estalibshed between the main flud path component 110 and the access port 350 since the opening defined in the distal end 342 of the valve element 340 is not flush or in contact with the inner sidewall 332 of the valve port 330.

Referring again to FIGS. 6-9, the valve element 340 may be threaded into the valve port 330 such the rotating the valve element 340 moves the valve element 340 along (i.e. parallel to) the valve axis 336. In particular, rotating the valve element 340 in one direction moves the valve element 340 from the open position *(see* FIGS. 7 and 9) to the closed position *(see* FIGS. 6 and 8), while rotating the valve element 340 in an opposite direction moves the valve element 340 from the closed position to the open position.

The valve element 340 may include an external threaded portion 346 located proximally of the distal end 342. The external threaded portion 346 engages a complementary internal threaded portion 372 of the valve port 330. In particular, the internal threaded portion 372 may be provided on a threaded insert 370 disposed in the valve port 330. The threaded insert 370 may be connected to the valve port 330 via a press fit, snap fit, adhesive, weld, or the like. In addition to allowing rotation of the valve element 340, the threaded insert 370 may also retain the seal in position in the valve port 330. It is also contemplated that the valve element 340 could be moved by a spring of a magnetic, electric, or pneumatic actuator.

## Claims

1. A valve (100) for use in an extracorporeal blood circulation system, the valve (100) comprising:
a main fluid path component (110) defining a longitudinal axis (112) and a main lumen (114) extending parallel to the longitudinal axis (112);
a valve port (130) defining a valve lumen (134);
an access port (150) extending at an angle from the valve lumen (134) and defining an access lumen (152) in fluid communication with the valve lumen (134); and
a valve element (140) disposed in the valve port (130) and movable between:
a closed position in which the valve element (140) engages a sidewall (132) of the valve lumen (134) to prevent fluid flow through the access lumen (152); and
an open position in which the valve element (140) is retracted relative to the closed position to allow fluid to flow through the valve lumen (134) and into the access lumen (152), and **characterized in that**
the valve port (130) has a tapered sidewall (132) narrowing in a direction toward the main lumen (114); and
wherein the valve element (140) comprises a tapered pin configured to seal against the tapered sidewall (132) in the closed position.

2. The valve (100) of claim 1, wherein the tapered sidewall (132) has a taper angle in a range of 3° to 15°.

3. The valve (100) of claim 1, wherein the tapered sidewall (132) acts as a stop preventing the tapered pin from protruding into the main lumen (114), or
wherein, in the closed position of the valve element (140), a distal end (142) of the valve element (140) is substantially flush with an inner sidewall (124) of the main fluid path component (110).

4. The valve (100) of claim 1, wherein the valve element (140) comprises a knob (148) for adjusting the valve element (140) between the closed position and the open position.

5. The valve (100) of claim 1, wherein the valve port (130) comprises a threaded section (172), and
wherein a proximal end of the valve element (140) is threaded to engage the threaded section (172) of the valve port (130), wherein optionally the threaded section (172) comprises a threaded insert (170) connected to the valve port (130).

6. The valve (100) of claim 1, wherein the valve port (130) extends at an angle of approximately 90° from a main fluid path component (110), or
wherein the access port (150) extends at angle from the valve port (130) in a range of approximately 30° to approximately 90°

7. The valve (100) of claim 1, wherein the access port (150) comprises a luer fitting, or
wherein the valve (100) further comprises a seal (160) disposed in the valve port (130) for preventing fluid flow between the valve element (140) and a sidewall (132) of the valve port (130), or
wherein the extracorporeal blood circulation system is selected from a group consisting of an ECMO machine, a heart-lung machine, a cardiopulmonary bypass machine, a pump-assisted lung protection machine, and a dialysis machine.

8. The valve (100) of any preceding claim, wherein the valve lumen (134) has a frusto-conical shape and the tapered pin of the valve element (140) has a complementary frusto-conical shape.

9. An extracorporeal blood circulation system, comprising:
a fluid path component (200); and
a valve (100) according to any of claims 1 to 8,
wherein the valve port (130) extends at an angle from the main lumen (114), and wherein the valve lumen (134) is in fluid communication with the main lumen (114), and
wherein the main fluid path component (110) is disposed internally in a fluid path of the fluid path component (200).

10. The system of claim 9, wherein the fluid path component (200) comprises at least one of an oxygenator and a heat exchanger.

11. The system of claim 9, wherein the extracorporeal blood circulation system is selected from a group consisting of an ECMO machine, a heart-lung machine, a cardiopulmonary bypass machine, a pump-assisted lung protection machine, and a dialysis machine.

12. A valve (300) for use in an extracorporeal blood circulation system, the valve (300) comprising:
a main fluid path component (110) defining a longitudinal axis (112) and a main lumen (114) extending parallel to the longitudinal axis (112);
a valve port (330) defining a valve lumen (334) in fluid communication with the main lumen (114);
a valve element (340) disposed in the valve port (330),
an access port (350) formed on a proximal end of the valve element (340) and defining an access lumen in fluid communication with the valve lumen (334), and
wherein the valve element (340) defines a fluid path channel (360) and is configured to be movable between:
a closed position in which the valve element (340) engages a sidewall (332) of the valve lumen (334) to prevent fluid flow through the access lumen; and
an open position in which the valve element (340) is retracted relative to the closed position to allow fluid to flow through the valve lumen (334) and into the access lumen; and **characterized in that**
the valve port (330) has a tapered sidewall narrowing in a direction toward the main lumen (114); and
wherein the valve element (340) comprises a tapered pin configured to seal against the tapered sidewall (332) in the closed position.

13. The valve (300) of claim 12, wherein the access port comprises a luer lock, or
wherein the fluid path channel (360) extends from a distal end (342) of the valve element (340) to a proximal end of the valve element (340).

14. The valve (300) of claim 12, wherein the tapered sidewall (332) has a taper angle in a range of 3° to 15°, or
wherein the tapered sidewall (332) acts as a stop preventing the tapered pin from protruding into the main path fluid component (110), or
wherein, in the closed position of the valve element (340), a distal end (342) of the valve element (340) is substantially flush with an inner sidewall (124) of the main fluid path component (110), or
wherein the valve (300) further comprises a seal disposed in the valve port (330) for preventing fluid flow between the valve element (340) and a sidewall (332) of the valve port (330), or
wherein the extracorporeal blood circulation system is selected from a group consisting of an ECMO machine, a heart-lung machine, a cardiopulmonary bypass machine, a pump-assisted lung protection machine, and a dialysis machine.

15. The valve (300) of any one of claims 12 to 14, wherein the valve lumen (334) has a frusto-conical shape and the tapered pin of the valve element (340) has a complementary frusto-conical shape.

## Patentansprüche

1. Ventil (100) zur Verwendung in einem extrakorporalen Blutkreislaufsystem, wobei das Ventil (100) Folgendes umfasst:
eine Hauptfluidwegkomponente (110), die eine Längsachse (112) und ein Hauptlumen (114), das sich parallel zu der Längsachse (112) erstreckt, definiert;
eine Ventilöffnung (130), die ein Ventillumen (134) definiert;
eine Zugangsöffnung (150), die sich in einem Winkel von dem Ventillumen (134) erstreckt und ein Zugangslumen (152) in Fluidkommunikation mit dem Ventillumen (134) definiert; und
ein Ventilelement (140), das in der Ventilöffnung (130) angeordnet und bewegbar ist zwischen:
einer geschlossenen Position, in der das Ventilelement (140) in eine Seitenwand (132) des Ventillumens (134) eingreift, um einen Fluidstrom durch das Zugangslumen (152) zu verhindern; und
einer offenen Position, in der das Ventilelement (140) gegenüber der geschlossenen Position zurückgezogen ist, um es einem Fluid zu ermöglichen, durch das Ventillumen (134) und in das Zugangslumen (152) zu strömen, und **dadurch gekennzeichnet, dass**
die Ventilöffnung (130) eine konische Seitenwand (132) aufweist, die sich in einer Richtung des Hauptlumens (114) verengt; und
wobei das Ventilelement (140) einen konischen Stift umfasst, der dazu konfiguriert ist, in der geschlossenen Position gegen die konische Seitenwand (132) abzudichten.

2. Ventil (100) nach Anspruch 1, wobei die konische Seitenwand (132) einen Konuswinkel in einem Bereich von 3° bis 15° aufweist.

3. Ventil (100) nach Anspruch 1, wobei die konische Seitenwand (132) als ein Anschlag fungiert, der den konischen Stift dran hindert, in das Hauptlumen (114) hineinzuragen, oder
wobei in der geschlossenen Position des Ventilelements (140) ein distales Ende (142) des Ventilelements (140) im Wesentlichen bündig ist mit einer inneren Seitenwand (124) der Hauptfluidwegkomponente (110).

4. Ventil (100) nach Anspruch 1, wobei das Ventilelement (140) einen Drehknopf (148) zum Einstellen des Ventilelements (140) zwischen der geschlossenen Position und der offenen Position umfasst.

5. Ventil (100) nach Anspruch 1, wobei die Ventilöffnung (130) einen Gewindeabschnitt (172) umfasst und
wobei ein proximales Ende des Ventilelements (140) ein Gewinde ist, um in den Gewindeabschnitt (172) der Ventilöffnung (130) einzugreifen, wobei der Gewindeabschnitt (172) optional einen Gewindeeinsatz (170) umfasst, der mit der Ventilöffnung (130) verbunden ist.

6. Ventil (100) nach Anspruch 1, wobei sich die Ventilöffnung (130) in einem Winkel von ungefähr 90° von einer Hauptfluidwegkomponente (110) erstreckt oder
wobei sich die Zugangsöffnung (150) in einem Winkel von der Ventilöffnung (130) in einem Bereich von ungefähr 30° bis ungefähr 90° erstreckt.

7. Ventil (100) nach Anspruch 1, wobei die Ventilöffnung (150) einen Luer-Anschluss umfasst oder
wobei das Ventil (100) ferner eine Dichtung (160) umfasst, die in der Ventilöffnung (130) zum Verhindern eines Fluidstroms zwischen dem Ventilelement (140) und einer Seitenwand (132) der Ventilöffnung (130) angeordnet ist, oder
wobei das extrakorporale Blutkreislaufsystem ausgewählt ist aus einer Gruppe bestehend aus einer ECMO-Maschine, einer Herz-Lungen-Maschine, einer kardiopulmonalen Bypass-Maschine, einer pumpengestützten Lungenschutzmaschine und einer Dialysemaschine.

8. Ventil (100) nach einem der vorhergehenden Ansprüche, wobei das Ventillumen (134) eine Kegelstumpfform aufweist und der konische Stift des Ventilelements (140) eine komplementäre Kegelstumpfform aufweist.

9. Extrakorporales Blutkreislaufsystem, umfassend:
eine Fluidwegkomponente (200); und
ein Ventil (100) nach einem der Ansprüche 1 bis 8,
wobei sich die Ventilöffnung (130) in einem Winkel von dem Hauptlumen (114) erstreckt und wobei das Ventillumen (134) in Fluidkommunikation mit dem Hauptlumen (114) steht und
wobei die Hauptfluidwegkomponente (110) intern in einem Fluidweg der Fluidwegkomponente (200) angeordnet ist.

10. System nach Anspruch 9, wobei die Fluidwegkomponente (200) mindestens eines von einem Oxygenator und einem Wärmetauscher umfasst.

11. System nach Anspruch 9, wobei das extrakorporale Blutkreislaufsystem ausgewählt ist aus einer Gruppe bestehend aus einer ECMO-Maschine, einer Herz-Lungen-Maschine, einer kardiopulmonalen Bypass-Maschine, einer pumpengestützten Lungenschutzmaschine und einer Dialysemaschine.

12. Ventil (300) zur Verwendung in einem extrakorporalen Blutkreislaufsystem, wobei das Ventil (300) Folgendes umfasst:
eine Hauptfluidwegkomponente (110), die eine Längsachse (112) und ein Hauptlumen (114), das sich parallel zu der Längsachse (112) erstreckt, definiert;
eine Ventilöffnung (330), die ein Ventillumen (334) in Fluidkommunikation mit dem Hauptlumen (114) definiert;
ein Ventilelement (340), das in der Ventilöffnung (330) angeordnet ist,
eine Zugangsöffnung (350), die an einem proximalen Ende des Ventilelements (340) gebildet ist und ein Zugangslumen in Fluidkommunikation mit dem Ventillumen (334) definiert, und
wobei das Ventilelement (340) einen Fluidwegkanal (360) definiert und dazu konfiguriert ist bewegbar zu sein zwischen:
einer geschlossenen Position, in der das Ventilelement (340) in eine Seitenwand (332) des Ventillumens (334) eingreift, um einen Fluidstrom durch das Zugangslumen zu verhindern; und
einer offenen Position, in der das Ventilelement (340) gegenüber der geschlossenen Position zurückgezogen ist, um es einem Fluid zu ermöglichen, durch das Ventillumen (334) und in das Zugangslumen zu strömen; und **dadurch gekennzeichnet, dass**
die Ventilöffnung (330) eine konische Seitenwand aufweist, die sich in einer Richtung des Hauptlumens (114) verengt; und
wobei das Ventilelement (340) einen konischen Stift umfasst, der dazu konfiguriert ist, in der geschlossenen Position gegen die konische Seitenwand (332) abzudichten.

13. Ventil (300) nach Anspruch 12, wobei die Zugangsöffnung einen Luer-Verschluss umfasst oder
wobei sich der Fluidwegkanal (360) von einem distalen Ende (342) des Ventilelements (340) zu einem proximalen Ende des Ventilelements (340) erstreckt.

14. Ventil (300) nach Anspruch 12, wobei die konische Seitenwand (332) einen Konuswinkel in einem Bereich von 3° bis 15° aufweist oder
wobei die konische Seitenwand (332) als ein Anschlag fungiert, der den konischen Stift dran hindert, in die Hauptwegfluidkomponente (110) hineinzuragen, oder
wobei in der geschlossenen Position des Ventilelements (340) ein distales Ende (342) des Ventilelements (340) im Wesentlichen bündig ist mit einer inneren Seitenwand (124) der Hauptfluidwegkomponente (110) oder
wobei das Ventil (300) ferner eine Dichtung umfasst, die in der Ventilöffnung (330) zum Verhindern eines Fluidstroms zwischen dem Ventilelement (340) und einer Seitenwand (332) der Ventilöffnung (330) angeordnet ist, oder
wobei das extrakorporale Blutkreislaufsystem ausgewählt ist aus einer Gruppe bestehend aus einer ECMO-Maschine, einer Herz-Lungen-Maschine, einer kardiopulmonalen Bypass-Maschine, einer pumpengestützten Lungenschutzmaschine und einer Dialysemaschine.

15. Ventil (300) nach einem der Ansprüche 12 bis 14, wobei das Ventillumen (334) eine Kegelstumpfform aufweist und der konische Stift des Ventilelements (340) eine komplementäre Kegelstumpfform aufweist.

## Revendications

1. Valve (100) pour l'utilisation dans un système de circulation sanguine extracorporelle, la valve (100) comprenant :
un composant de trajet de fluide principal (110) définissant un axe longitudinal (112) et une lumière principale (114) s'étendant parallèlement à l'axe longitudinal (112) ;
un orifice de valve (130) définissant une lumière de valve (134) ;
un orifice d'accès (150) s'étendant à un angle à partir de la lumière de valve (134) et définissant une lumière d'accès (152) en communication fluidique avec la lumière de valve (134) ; et
un élément de valve (140) disposé dans l'orifice de valve (130) et mobile entre :
une position fermée dans laquelle l'élément de valve (140) met en prise une paroi latérale (132) de la lumière de valve (134) pour la prévention d'un écoulement de fluide à travers la lumière d'accès (152) ; et
une position ouverte dans laquelle l'élément de valve (140) est rétracté par rapport à la position fermée pour la permission à un fluide de s'écouler à travers la lumière de valve (134) et à l'intérieur de la lumière d'accès (152), et **caractérisée en ce que**
l'orifice de valve (130) a une paroi latérale effilée (132) se rétrécissant dans une direction vers la lumière principale (114) ; et
dans laquelle l'élément de valve (140) comprend une broche effilée configurée pour l'étanchéité contre la paroi latérale effilée (132) dans la position fermée.

2. Valve (100) selon la revendication 1, dans laquelle la paroi latérale effilée (132) a un angle d'effilement dans une plage de 3° à 15°.

3. Valve (100) selon la revendication 1, dans laquelle la paroi latérale effilée (132) agit comme une butée empêchant la broche effilée de faire saillie à l'intérieur de la lumière principale (114), ou
dans laquelle, dans la position fermée de l'élément de valve (140), une extrémité distale (142) de l'élément de valve (140) est sensiblement affleurante avec une paroi latérale interne (124) du composant de trajet de fluide principal (110).

4. Valve (100) selon la revendication 1, dans laquelle l'élément de valve (140) comprend un bouton (148) pour l'ajustement de l'élément de valve (140) entre la position fermée et la position ouverte.

5. Valve (100) selon la revendication 1, dans laquelle l'orifice de valve (130) comprend une section filetée (172), et
dans laquelle une extrémité proximale de l'élément de valve (140) est filetée pour la mise en prise de la section filetée (172) de l'orifice de valve (130), dans laquelle facultativement la section filetée (172) comprend un insert fileté (170) raccordé à l'orifice de valve (130).

6. Valve (100) selon la revendication 1, dans laquelle l'orifice de valve (130) s'étend à un angle d'approximativement 90° à partir d'un composant de trajet de fluide principal (110), ou
dans laquelle l'orifice d'accès (150) s'étend à un angle à partir de l'orifice de valve (130) dans une plage d'approximativement 30° à approximativement 90°.

7. Valve (100) selon la revendication 1, dans laquelle l'orifice d'accès (150) comprend un raccord luer, ou
dans laquelle la valve (100) comprend en outre un joint d'étanchéité (160) disposé dans l'orifice de valve (130) pour la prévention d'un écoulement de fluide entre l'élément de valve (140) et une paroi latérale (132) de l'orifice de valve (130), ou
dans laquelle le système de circulation sanguine extracorporelle est sélectionné dans un groupe constitué d'une machine d'ECMO, d'une machine cœur-poumons, d'une machine de pontage cardiopulmonaire, d'une machine de protection pulmonaire assistée par pompe et d'une machine de dialyse.

8. Valve (100) selon l'une quelconque des revendications précédentes, dans laquelle la lumière de valve (134) a une forme tronconique et la broche effilée de l'élément de valve (140) a une forme tronconique complémentaire.

9. Système de circulation sanguine extracorporelle, comprenant :
un composant de trajet de fluide (200) ; et
une valve (100) selon l'une quelconque des revendications 1 à 8,
dans lequel l'orifice de valve (130) s'étend à un angle à partir de la lumière principale (114), et dans lequel la lumière de valve (134) est en communication fluidique avec la lumière principale (114), et
dans lequel le composant de trajet de fluide principal (110) est disposé de manière interne dans un trajet de fluide du composant de trajet de fluide (200).

10. Système selon la revendication 9, dans lequel le composant de trajet de fluide (200) comprend au moins l'un parmi un oxygénateur et un échangeur de chaleur.

11. Système selon la revendication 9, dans lequel le système de circulation sanguine extracorporelle est sélectionné dans un groupe constitué d'une machine d'ECMO, d'une machine cœur-poumons, d'une machine de pontage cardiopulmonaire, d'une machine de protection pulmonaire assistée par pompe et d'une machine de dialyse.

12. Valve (300) pour l'utilisation dans un système de circulation sanguine extracorporelle, la valve (300) comprenant :
un composant de trajet de fluide principal (110) définissant un axe longitudinal (112) et une lumière principale (114) s'étendant parallèlement à l'axe longitudinal (112) ;
un orifice de valve (330) définissant une lumière de valve (334) en communication fluidique avec la lumière principale (114) ;
un élément de valve (340) disposé dans l'orifice de valve (330),
un orifice d'accès (350) formé sur une extrémité proximale de l'élément de valve (340) et définissant une lumière d'accès en communication fluidique avec la lumière de valve (334), et
dans laquelle l'élément de valve (340) définit un canal de trajet de fluide (360) et est configuré pour être mobile entre :
une position fermée dans laquelle l'élément de valve (340) met en prise une paroi latérale (332) de la lumière de valve (334) pour la prévention d'un écoulement de fluide à travers la lumière d'accès ; et
une position ouverte dans laquelle l'élément de valve (340) est rétracté par rapport à la position fermée pour la permission à un fluide de s'écouler à travers la lumière de valve (334) et à l'intérieur de la lumière d'accès ; et **caractérisée en ce que**
l'orifice de valve (330) a une paroi latérale effilée se rétrécissant dans une direction vers la lumière principale (114) ; et
dans laquelle l'élément de valve (340) comprend une broche effilée configurée pour l'étanchéité contre la paroi latérale effilée (332) dans la position fermée.

13. Valve (300) selon la revendication 12, dans laquelle l'orifice d'accès comprend un verrou luer, ou
dans laquelle le canal de trajet de fluide (360) s'étend à partir d'une extrémité distale (342) de l'élément de valve (340) vers une extrémité proximale de l'élément de valve (340).

14. Valve (300) selon la revendication 12, dans laquelle la paroi latérale effilée (332) a un angle d'effilement dans une plage de 3° à 15°, ou
dans laquelle la paroi latérale effilée (332) agit comme une butée empêchant la broche effilée de faire saillie à l'intérieur du composant de fluide de trajet principal (110), ou
dans laquelle, dans la position fermée de l'élément de valve (340), une extrémité distale (342) de l'élément de valve (340) est sensiblement affleurante avec une paroi latérale interne (124) du composant de trajet de fluide principal (110), ou
dans laquelle la valve (300) comprend en outre un joint d'étanchéité disposé dans l'orifice de valve (330) pour la prévention d'un écoulement de fluide entre l'élément de valve (340) et une paroi latérale (332) de l'orifice de valve (330), ou
dans laquelle le système de circulation sanguine extracorporelle est sélectionné dans un groupe constitué d'une machine d'ECMO, d'une machine cœur-poumons, d'une machine de pontage cardiopulmonaire, d'une machine de protection pulmonaire assistée par pompe et d'une machine de dialyse.

15. Valve (300) selon l'une quelconque des revendications 12 à 14, dans laquelle la lumière de valve (334) a une forme tronconique et la broche effilée de l'élément de valve (340) a une forme tronconique complémentaire.
